(19) Europäisches Patentamt
European Patent Office
Office européen des brevets

(11) **EP 3 511 053 B1**

(12) **EUROPÄISCHE PATENTSCHRIFT**

(45) Veröffentlichungstag und Bekanntmachung des Hinweises auf die Patenterteilung:
**04.03.2020  Patentblatt 2020/10**

(51) Int Cl.:
***A61N 5/10*** *(2006.01)*

(21) Anmeldenummer: **18150964.7**

(22) Anmeldetag: **10.01.2018**

(54) **VERFAHREN UND VORRICHTUNG ZUR BESTIMMUNG DES BREMSVERMÖGENS FÜR DIE PROTONENTHERAPIE**

METHOD AND DEVICE FOR DETERMINING BRAKING POTENTIAL FOR PROTON THERAPY

PROCÉDÉ ET DISPOSITIF DE DÉTERMINATION DE LA PUISSANCE DE FREINAGE POUR UNE THÉRAPIE PROTONIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**17.07.2019  Patentblatt 2019/29**

(73) Patentinhaber: **Siemens Healthcare GmbH 91052 Erlangen (DE)**

(72) Erfinder: **Hofmann, Christian 91052 Erlangen (DE)**

(56) Entgegenhaltungen:
- **Christian Möhler et al: "Experimental verification of stopping-power prediction from single- and dual-energy computed tomography in biological tissues", Phys. Med. Biol., Bd. 63, Nr. 2 9. Januar 2018 (2018-01-09), Seite 17PP, XP002783120, Gefunden im Internet: URL:https://arxiv.org/ftp/arxiv/papers/170 8/1708.07368.pdf [gefunden am 2018-07-17]**
- **Nora Hünemohr et al: "Ion range estimation by using dual energy computed tomography", Zeitschrift für Medizinische Physik, Bd. 23, Nr. 4 15. April 2013 (2013-04-15), Seiten 300-313, XP002783121, Gefunden im Internet: URL:https://ac.els-cdn.com/S09393889130003 17/1-s2.0-S0939388913000317-main.pdf?_tid= 92b8e6fa-7eb0-41cd-9eac-ba12781d01b7&acdna t=1531833618_3ebc8c18c16b68e3c6fc576fe7ed 2 be6 [gefunden am 2018-07-17]**
- **NORA HÜNEMOHR ET AL: "Experimental verification of ion stopping power prediction from dual energy CT data in tissue surrogates", PHYSICS IN MEDICINE AND BIOLOGY, INSTITUTE OF PHYSICS PUBLISHING, Bd. 59, Nr. 1, 12. Dezember 2013 (2013-12-12), Seiten 83-96, XP020255808, BRISTOL GB ISSN: 0031-9155, DOI: 10.1088/0031-9155/59/1/83 [gefunden am 2013-12-12]**

**Beschreibung**

Die Erfindung betrifft ein Verfahren und eine Vorrichtung zur Bestimmung des Bremsvermögens für die Partikeltherapie, insbesondere für die Protonentherapie. Die Erfindung betrifft des Weiteren eine entsprechende Steuereinrichtung für ein Computertomographiesystem und ein Computertomographiesystem umfassend eine solche Steuereinrichtung.

Heutzutage wird das Bremsvermögen ("SPR" für engl.: "Stopping Power Ratio") von Protonen für die Behandlungsplanung einer Protonentherapie in der Regel aus Single-Energy-Daten einer Computertomographie-Aufnahme gewonnen. Dabei bezieht sich der Begriff "Single-Energy" ("Eine Energie") auf die Art und Weise der Computertomographie-Aufnahme mit nur einer einzigen Aufnahmeenergie. Aus der rekonstruierten Computertomographie-Aufnahme oder ihren Rohdaten werden dabei für die einzelnen Voxel sogenannte "CT-Werte" ermittelt. Diesen CT-Werten werden dann SPR-Werte zugeordnet, also Werte, die das Bremsvermögen wiedergeben. Die Natur der CT-Werte, die im Folgenden auch als "CT-Zahlen" bezeichnet werden, sind dem Fachmann auf diesem Gebiet bekannt. Sie liegen in der Regel in Hounsfield-Einheiten H vor.

Die Zuordnung von SPR-Werten zu CT-Zahlen kann mittels einer Nachschlagetabelle (engl.: "Look-up-table" bzw. "Hounsfield-look-up-table"; HLUT) erreicht werden. Basierend auf Kalibrierungsmessungen wird eine solche Nachschlagetabelle für Single-Energy-Daten generiert, die einem CT-Wert einen diskreten SPR-Wert zuweist.

Solche Kalibrierungsmessungen stellen einen Nachteil dar, da sie einen zusätzlichen Aufwand und eine zusätzliche potentielle Fehlerquelle bedeuten.

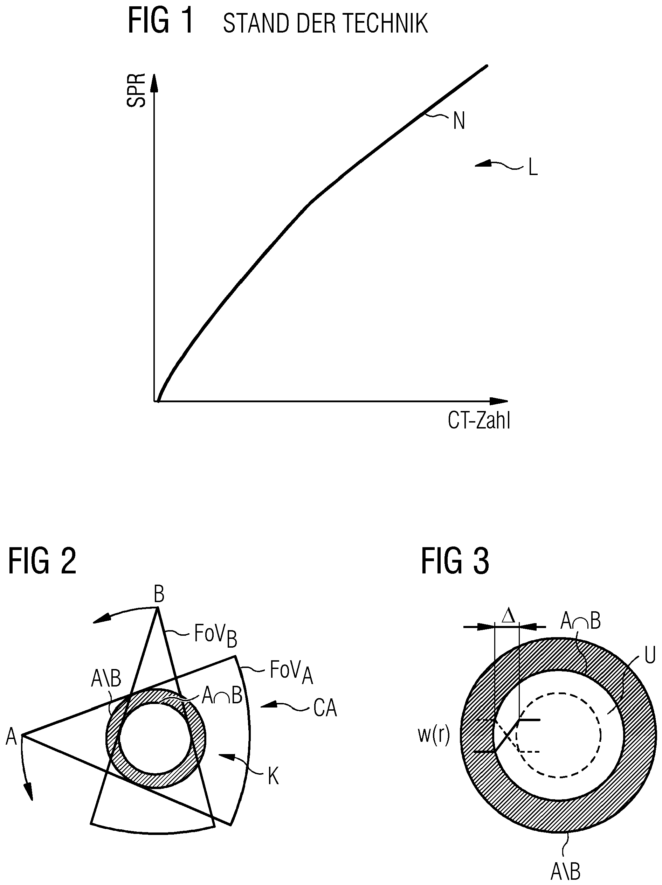

Es ist allgemein üblich, je nach Behandlung unterschiedliche Nachschlagetabellen zu generieren, z.B. eine für Behandlungen des Kopfes und eine für Behandlungen des Körpers. Eine beispielhafte Nachschlagetabelle ist in Figur 1 dargestellt.

In der letzten Zeit wurde die Nutzung von Dual-Energy-CT-Aufnahmen ("DECT") als eine Alternative zu dem vorangehenden Ansatz entdeckt. Dabei bezieht sich der Begriff "Dual-Energy" ("Zwei Energien") ebenfalls auf die Art und Weise des Aufnahmeprinzips mit zwei unterschiedlichen Aufnahmeenergien. Gemäß diesem Prinzip wird zur Ermittlung des Bremsvermögens SPR die spektrale Information der Dual-Energy-Daten verwendet, womit SPR-Werte direkt aus CT-Zahlen berechnet werden können (siehe z.B. "Ion Range estimation by using dual energy computed tomography"; N. Hünemohr et. al.; Z. Med. Phys. 23 (2013) 300-313 oder ""Experimental verification of ion stopping power prediction from dual energy CT data in tissue surrogates"; N. Hünemohr et. al.; Phys. Med. Biol. 59 (2014) 83-96).

Es gibt Dual-Energy-CT-Systeme auf dem Markt, welche die Bilder mit hoher und niedriger Energie mit zwei verschiedenen Sichtfeldern (engl.: Field of view: "FOV") aufnehmen. Die Folge davon ist eine räumliche Einschränkung der Vorhersage für das SPR, da Dual-Energy-Daten nur innerhalb des kleineren Sichtfeldes (FOV) möglich ist.

Diese räumliche Einschränkung stellt einen Nachteil für die Behandlungsplanung der Protonentherapie dar, da für diejenigen Bereiche, für die nur Single-Energy-Daten vorhanden sind, wieder Kalibrierungsdaten aufgenommen werden müssen, die zu den weiter oben genannten Nachteilen führen.

Es ist eine Aufgabe der vorliegenden Erfindung, ein alternatives, komfortableres Verfahren und eine entsprechende Vorrichtung und ferner eine Steuereinrichtung zur Steuerung eines Computertomographiesystems oder eines Partikeltherapiesystems und ein entsprechendes Computertomographiesystem bzw. Partikeltherapiesystem zur Verfügung zu stellen, mit dem die oben beschriebenen Nachteile vermieden werden.

Diese Aufgabe wird durch ein Verfahren gemäß Patentanspruch 1, eine Vorrichtung gemäß Patentanspruch 10 sowie durch eine Steuereinrichtung gemäß Patentanspruch 11 und ein Computertomographiesystem gemäß Patentanspruch 12 oder eines Partikeltherapiesystems gemäß Patentanspruch 13 gelöst.

Das erfindungsgemäße Verfahren dient zur Ermittlung des Bremsvermögens ("SPR") aus Computertomographie-Aufnahmen für den technischen Bereich der Partikeltherapie, insbesondere der Protonentherapie. Es umfasst die folgenden die Schritte.

- Bereitstellung von Computertomographie-Aufnahmen

Diese Bereitstellung umfasst dabei sowohl die Bereitstellung von bereits vorhandenen Computertomographie-Aufnahmen als auch die Anfertigung neuer Computertomographie-Aufnahmen, also z.B. eine direkte Aufnahme durch einen Computertomographen. Da die Aufnahme mit zwei Aufnahmeenergien erfolgte, werden die Computertomographie-Aufnahmen werden auch als "Dual-Energy-CT Aufnahmen" (DECT-Aufnahmen) bezeichnet. Zur Aufnahme können z.B. zwei Bildaufnahmeeinheiten eines Computertomographen ("CT") über einen Winkelbereich von mindestens 90° bevorzugt mindestens 180° um den Körperbereich herumgeschwenkt werden und währenddessen eine Anzahl von mehreren Bildern anfertigen.

Die Computertomographie-Aufnahmen stellen dabei einen Körperbereich dar und enthalten aufgrund der unterschiedlichen Sichtbereiche ("FoV") für unterschiedliche Aufnahmeenergien partiell Bildinformationen über zwei unterschiedliche Aufnahmeenergien, mit denen dieser Körperbereich aufgenommen worden ist. Diese Bereiche werden als "Dual-Energy-Bildbereiche" bezeichnet, in Unterschied zu denjenigen Bereichen, in denen aufgrund der unterschiedlichen Sichtbereiche nur Informationen für eine einzige Aufnahmeenergie vorliegen. Diese werden als "Single-Energy-Bildbereiche" bezeichnet.

Normalerweise enthalten die (rekonstruierten) Aufnahmen Informationen über CT-Zahlen, so dass in der Regel jedem Voxel eine CT-Zahl zugeordnet ist. Sollte dies nicht der Fall sein, weil dem Verfahren z.B. nur Rohdaten eines CT zur Verfügung stehen, würden die CT-Zahlen H in diesem Schritt auch noch aus den Bilddaten der Computertomographie-Aufnahmen berechnet werden. Dies kann beispielsweise in einem Fall geschehen, in dem der Schwächungskoeffizient $\mu$ eines Voxels und der Schwächungskoeffizient von Wasser $\mu_W$ und von Luft $\mu_L$ bekannt ist mit der Formel

$$H = \frac{\mu - \mu_W}{\mu_W - \mu_{Luft}} \cdot 1000 HU \qquad (1)$$

erfolgen, wobei die Zahl 1000 in der Regel verwendet wird, aber theoretisch auch Faktoren mit einem anderen Wert verwendet werden können. Es könnte auch zur Vereinfachung ein $\mu_L$ mit dem Wert = 0 angenommen werden.

- Ermittlung des Bremsvermögens $SPR_{DE}$

In diesem Schritt wird für unterschiedliche CT-Zahlen H das Bremsvermögen $SPR_{DE}$ aus Dual-Energy-Bildbereichen der Computertomographie-Aufnahmen ermittelt, also denjenigen Bildbereichen, in denen Bilddaten mit beiden Aufnahmeenergien vorliegen. Die Rechenschritte zur Ermittlung des Bremsvermögens $SPR_{DE}$ aus DECT-Daten sind wohlbekannt, wobei mehrere mögliche Wege existieren. Dennoch ist ein konkretes Beispiel zu jedem dieser Wege so komplex, dass es den Rahmen dieser Beschreibung sprengen würde. Daher wird hier lediglich auf den Stand der Technik zu der Ermittlung des Bremsvermögens SPR aus DECT-Daten verwiesen, z.B. auf die in der Einleitung genannten Veröffentlichungen.

- Bildung eines Datenfeldes

Die im vorangegangenen Schritt ermittelten Werte für das Bremsvermögen $SPR_{DE}$ für unterschiedliche CT-Zahlen H aus Dual-Energy-Bildbereichen werden in Abhängigkeit zu den CT-Zahlen H in einem Datenfeld aufgetragen. Dabei enthält das Datenfeld in der Regel für eine einzige CT-Zahl nicht einen einzigen Wert für das Bremsvermögen $SPR_{DE}$. Umfasst diese Auftragung Bins, die mit den Werten gefüllt werden, erhält man in der Regel eine Verteilung von Werten die zumeist ein Extremum aufweist. Sofern das Bremsvermögen $SPR_{DE}$, mit den wohlbekannten Verfahren ermittelt worden ist, handelt es sich bei dem Extremum in der Regel um ein Maximum.

- Bildung einer Nachschlagetabelle

Aus dem im vorangegangenen Schritt erhaltenen Datenfeld wird nun eine Nachschlagetabelle gebildet. Eine solche Nachschlagetabelle wird auch als HLUT ("Hounsfield Look Up Table") bezeichnet. Dabei sind zur Bildung der Nachschlagetabelle keine Kalibrationsmessungen notwendig. Der Erfinder hat erkannt, dass die Nachschlagetabelle direkt aus dem Datenfeld gebildet werden kann. Konkrete Beispiele zur Bildung der Nachschlagetabelle werden weiter unten genauer ausgeführt.

- Ermittlung des Bremsvermögens $SPR_{SE}$

Mit der aus dem vorangegangenen Schritt erhaltenen Nachschlagetabelle ist es nun möglich, für unterschiedliche CT-Zahlen H aus Single-Energy-Bildbereichen der Computertomographie-Aufnahmen, also den Bereichen, in denen Bilddaten mit nur einer einzigen Aufnahmeenergie vorliegen, das Bremsvermögen $SPR_{SE}$ zu ermitteln. Bei einer direkten CT-Aufnahme kann das Bremsvermögen $SPR_{SE}$ damit durch das Verfahren auch für Single-Energy-Bildbereiche in Echtzeit angegeben werden.

Eine erfindungsgemäße Vorrichtung zur Ermittlung des Bremsvermögens SPR aus Computertomographie-Aufnahmen umfasst die folgenden Komponenten.

- Eine Schnittstelle zum Empfang von Computertomographie-Aufnahmen eines Körperbereichs mit zwei unterschiedlichen Aufnahmeenergien. In dem Falle, dass diese Computertomographie-Aufnahmen keine CT-Zahlen umfassen, da sie z.B. als Rohdaten vorliegen, ist es von Vorteil, wenn diese Schnittstelle eine Einheit umfasst, welche diese CT-Zahlen aus den Bilddaten ermitteln kann, z.B. eine Bildrekonstruktionseinheit.

- Eine erste Ermittlungseinheit zur Ermittlung des Bremsvermögens $SPR_{DE}$ für unterschiedliche CT-Zahlen H aus Dual-Energy-Bildbereichen der Computertomographie-Aufnahmen, wie vorangehend bereits genauer ausgeführt worden ist.

- Eine Recheneinheit zur Bildung eines Datenfeldes, in dem die ermittelten Werte für das Bremsvermögen $SPR_{DE}$ für unterschiedliche CT-Zahlen H aus Dual-Energy-Bildbereichen in Abhängigkeit zu den CT-Zahlen H aufgetragen sind, wie ebenfalls vorangehend bereits genauer ausgeführt worden ist.

- Ein Nachschlagetabellen-Modul zur Bildung einer Nachschlagetabelle aus dem Datenfeld.

- Eine zweite Ermittlungseinheit zur Ermittlung des Bremsvermögens $SPR_{SE}$ für unterschiedliche CT-Zahlen H aus Single-Energy-Bereichen der Computertomographie-Aufnahmen mittels der Nachschlagetabelle.

Eine erfindungsgemäße Steuereinrichtung zur Steuerung eines Computertomographiesystems oder eines Partikeltherapiesystems, insbesondere Protonentherapiesystems, ist zur Durchführung des erfindungsgemäßen Verfahrens ausgelegt und/oder umfasst eine erfindungsgemäße Vorrichtung.

Ein erfindungsgemäßes Computertomographiesystem umfasst eine erfindungsgemäße Steuereinrichtung.

Ein erfindungsgemäßes Partikeltherapiesystem, insbesondere Protonentherapiesystem, umfasst eine erfindungsgemäße Steuereinrichtung.

Ein Großteil der zuvor genannten Komponenten der Vorrichtung oder der Steuereinrichtung, können ganz oder teilweise in Form von Softwaremodulen in einem Prozessor eines Rechensystems bzw. einer entsprechenden Steuereinrichtung realisiert werden. Eine weitgehend softwaremäßige Realisierung hat den Vorteil, dass auch schon bisher verwendete Rechensysteme bzw. Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines Rechensystems bzw. einer Steuereinrichtung ladbar ist, mit Programmabschnitten, um alle Schritte des erfindungsgemäßen Verfahrens auszuführen, wenn das Programm in dem Rechensystem bzw. der Steuereinrichtung ausgeführt wird. Ein solches Computerprogrammprodukt kann neben dem Computerprogramm gegebenenfalls zusätzliche Bestandteile wie z.B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Rechensystem bzw. zur Steuereinrichtung und/oder zur Speicherung an oder in dem Rechensystem bzw. der Steuereinrichtung kann ein computerlesbares Medium, beispielsweise ein Memorystick, eine Festplatte oder ein sonstiger transportabler oder fest eingebauter Datenträger dienen, auf welchem die von dem Rechensystem bzw. einer Rechnereinheit der Steuereinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bevorzugt erfolgt die Bildung des Datenfeldes auf eine Weise, dass es als Punktwolke vorliegt, womit eine zweidimensionale Verteilung von Werten gemeint ist. In einem bevorzugten Datenfeld, bei dem die horizontale Achse die CT-Zahl H angibt und die vertikale Achse das Bremsvermögen SPR, liegen die ermittelten Werte des Bremsvermögens $SPR_{DE}$ aus den Double-Energy-Daten als eine zweidimensionale Werteverteilung (Punktwolke) in dem Datenfeld vor. Die Punktwolke kann beispielsweise eine Dichteverteilung aufweisen.

In dem bevorzugten Fall, dass die Werte in Form eines zweidimensionalen Histogramms aufgetragen werden, entspricht der CT-Wert H in der Regel der Spalte und der Wert für das Bremsvermögen SPR der Zeile eines Feldes mit diskreten Koordinaten (x, y), die Bins darstellen. Die Punkte liegen in diesem Fall in Form einer Häufigkeitsverteilung vor. Dies bedeutet, dass für eine Spalte des vorgenannten diskreten Feldes (eine CT-Zahl) eine Häufigkeitsverteilung von für diesen CT-Wert berechneten Bremsvermögen vorliegt. Die Gesamtheit aller Werte des Datenfeldes ergibt also eine Punktwolke, die eine zweidimensionale Häufigkeitsverteilung darstellt.

In diese Punktwolke wird eine Funktion angepasst. Das Anpassen einer Funktion ist dem Fachmann bekannt und wird auch als "Ausgleichungsrechnung" oder auch als "Kurvenfit" bezeichnet. Beispielsweise könnte man sich zur Anpassung der Funktion an die Punktwolke des Gauß-Newton-Verfahrens bedienen.

Die Funktion gibt für eine CT-Zahl einen diskreten Wert für das Bremsvermögen $SPR_L$ an, entspricht also einer Funktion $SPR_L$(CT-Zahl). Damit definiert die Funktion direkt Werte einer Nachschlagetabelle. Diese Nachschlagetabelle kann in Form einer konkreten Tabelle vorliegen, in der für jede CT-Zahl ein SPR-Wert direkt abgelesen werden kann.

Diesbezüglich würde die Funktion F lediglich einen Weg zum Erhalt der Werte darstellen. In einem anderen Fall kann auch ein Graph, an dem in einem Diagramm der SPR-Wert abgelesen werden kann, die Nachschlagetabelle darstellen. Letztendlich kann bei einer automatischen Bearbeitung auch die Funktion die Nachschlagetabelle darstellen, da diese jeder CT-Zahl einen SPR-Wert zuordnet.

Wenn eine CT-Zahl initial vorliegt, kann diese bevorzugt mittels der Funktion direkt in einen Wert für das Bremsvermögen $SPR_L$ umgerechnet werden, wobei in dem Falle, dass CT-Zahlen von Punkten aus Single-Energy-Bildbereichen der Computertomographie-Aufnahmen verwendet werden $SPR_L = SPR_{SE}$ gilt.

In diesem Sinne ist bei einer bevorzugten Vorrichtung die Recheneinheit dazu ausgelegt, ein Datenfeld in Form einer Punktwolke, insbesondere in Form eines zweidimensionalen Histogramms, zu bilden und das Nachschlagetabellen-Modul ist dazu ausgelegt, eine Funktion an die Punktwolke anzupassen, wobei die Funktion Werte der Nachschlagetabelle definiert.

Bevorzugt wird für eine Anzahl von Voxeln, insbesondere für alle Voxel, einer Computertomographie-Aufnahme das ortsabhängige Bremsvermögen $SPR(x, y, z)$ ermittelt. Dies geschieht entweder durch direkte Berechnung für Dual-Energy-Bildbereiche oder mittels der Nachschlagetabelle für Single-Energy-Bildbereiche. Dies kann dadurch erreicht werden, dass für die betreffenden Voxel, welche eine bekannte Bildposition $(x, y, z)$ haben, die Positionsinformationen bei der Berechnung des Bremsvermögens SPR übernommen werden. Die Z-Koordinate ist dabei bei zweidimensionalen Bilddaten nicht unbedingt notwendig, bei dreidimensionalen Bilddaten jedoch von großem Vorteil. Bei Auftragung der Bremsvermögen $SPR(x, y, z)$ an den entsprechenden Koordinaten ergibt sich ein Bild, dessen Bildinformationen für jeden Punkt des aufgenommenen Körperbereichs direkt das Bremsvermögen angibt. Hier kann SPR je nach Bildbereich dem Wert $SPR_{DE}$ oder $SPR_{SE}$ entsprechen.

Bevorzugt werden für unterschiedliche Durchmesser von aufgenommenen Regionen unterschiedliche Nachschlagetabellen erstellt. Damit können Effekte basierend auf einer Strahlaushärtung kompensiert werden, was die Genauigkeit der Ermittlung von Werten für das Bremsvermögen für unterschiedliche Körperbereiche deutlich verbessert. Beispielsweise können für vorher festgelegte Durchmesser, z.B. 10 cm, 20 cm, 30 cm, 40 cm, ..., unterschiedliche Nachschlagetabellen ermittelt werden.

Bevorzugt wird für diese Nachschlagetabellen der betreffende Durchmesser aus den betreffenden Computertomographie-Aufnahmen ermittelt oder geschätzt. Eine geeignete Schätzung des Durchmessers eines in einem Schnittbild abgebildeten Objekts kann beispielsweise mit einem Algorithmus zur differenziellen Evolution ("Differential Evolution Algorithm") erreicht werden.

Bevorzugt wird zur Ermittlung eines Wertes für das Bremsvermögen $SPR_{SE}$ für eine CT-Zahl im Single-Energy-Bereich der in diesem Bereich vorliegende Durchmesser geschätzt (z.B. mit dem vorgenannten Algorithmus) und aus einer linearen Interpolation von den Funktionen (bzw. Nachschlagewerten) von den vorgenannten Nachschlagetabellen benachbarter Durchmesser der in dem vorliegenden Fall anzunehmende Wert für das Bremsvermögen $SPR_{SE}$ ermittelt. Soll z.B. der Wert für das Bremsvermögen $SPR_{SE}$ in einem Single-Energy-Bereich an einer Stelle des Beines ermittelt werden, für die ein Durchmesser von 15 cm geschätzt worden ist, würden aus den vorgenannten Nachschlagetabellen diejenigen mit 10 cm Durchmesser und 20 cm Durchmesser als benachbart ausgewählt und deren jeweilige Funktionen (bzw. Nachschlagewerte) mittels linearer Interpolation auf den Durchmesser 15 cm angepasst werden. Selbstverständlich ist auch eine feinere Abtastung als in 10 cm Schritten möglich, wobei mit zunehmend feinerer Abtastung auch die linear interpolierten Werte immer genauer werden.

Bevorzugt erfolgt vor der Ermittlung der Werte des Bremsvermögens $SPR_{DE}$ eine Korrektur der Strahlaufhärtung (engl.: Beam Hardening. Dazu erfolgen bevorzugt dedizierte Kalibrationen, um dedizierte Lookup-Tabellen zu erzeugen. Dies hat den Vorteil, dass der spektralen Effekt der Strahlaufhärtung auf die aufgenommenen Bilder bereits vor der Ermittlung des Bremsvermögens kompensiert werden kann.

Bevorzugt wird im Falle aneinandergrenzender Dual-Energy- und Single-Energy-Bildbereiche die Wichtung des Einflusses der Bremsvermögen $SPR_{DE}$ des Dual-Energy-Bildbereichs und das Bremsvermögen $SPR_{SE}$ des Single-Energy-Bildbereichs bestimmt. Dies geschieht in einem vorbestimmten Übergangsbereich der Dicke $\Delta$ bevorzugt mittels einer vorbestimmten Übergangsfunktion $w(r)$ mit $0 < r < \Delta$. Der Wert $r$ stellt dabei ein räumliches Maß eines Teils der Dicke dar. Dies kann z.B. ein einfaches Maß (in mm oder Bildpunkten, d.h. Pixeln oder Voxeln) sein oder auch ein Bruchteil der Dicke.

Dazu wird aus dem Bremsvermögen $SPR_{DE}$ des Dual-Energy-Bildbereichs und dem Bremsvermögen $SPR_{SE}$ des Single-Energy-Bildbereichs bevorzugt ein effektives Bremsvermögen $SPR_{Eff}$ über den gesamten Übergangsbereich bestimmt. Dies geschieht besonders bevorzugt gemäß der Formel

$$SPR_{Eff}(r) = w(r) \cdot SPR_{DE} + (1 - w(r)) \cdot SPR_{SE} . \qquad (2)$$

Die Übergangsfunktion $w(r)$ kann beispielsweise für einen linearen Übergang mit $w(r) = \Delta - r$ gewählt werden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:

Figur 1 eine schematische Darstellung einer Nachschlagetabelle gemäß dem Stand der Technik,

Figur 2 eine Darstellung einer Computertomographie-Aufnahme mit zwei Energien und zwei Sichtbereichen FoV,

Figur 3 eine Skizzierung des Übergangsbereichs,

Figur 4 ein schematisches Blockbild des erfindungsgemäßen Verfahrens,

Figur 5 ein mittels der Erfindung erstelltes Datenfeld sowie eine mittels der Erfindung erstellte Nachschlagetabelle,

Figur 6 eine grob schematische Darstellung eines bildgebenden Systems mit einem Ausführungsbeispiel einer erfindungsgemäßen Steuereinrichtung zur Durchführung des Verfahrens.

Figur 7 eine grob schematische Darstellung eines Partikeltherapiesystems mit einem Ausführungsbeispiel einer erfindungsgemäßen Steuereinrichtung zur Durchführung des Verfahrens.

Figur 1 zeigt eine schematische Darstellung einer Nachschlagetabelle L gemäß dem Stand der Technik. Wie bereits im einleitenden Teil erklärt wurde. Basierend auf Kalibrierungsmessungen wird eine Nachschlagetabelle L für Single-Energy-Daten generiert. Die Nachschlagewert-Kurve N weist jedem CT-Wert in einem bestimmten Intervall einen SPR-Wert zu.

Figur 2 zeigt eine Darstellung einer Computertomographie-Aufnahme CA mit zwei Aufnahmeenergien A, B und zwei Sichtbereichen $FoV_A$, $FoV_B$. Die beiden als Kegel angedeuteten Sichtbereiche $FoV_A$, $FoV_B$ drehen sich dabei während der Aufnahme um einen bestimmten Winkelbereich, z.B. 180°, und nehmen dabei den Körperbereich K auf, der hier als Kreis dargestellt ist. Dabei überstreicht der Sichtbereich $FoV_A$ der Aufnahmeenergie A ein größeres Areal des Körperbereichs K als der Sichtbereich $FoV_B$ der Aufnahmeenergie B. Dadurch liegen im inneren Teil des Kreises, dem Dual-Energy-Bildbereich A∩B, Bilddaten mit beiden Aufnahmeenergien A, B vor und in dem äußeren Teil des Kreises, dem Single-Energy-Bildbereich (A\B), Bilddaten mit nur einer einzigen Aufnahmeenergie A vor.

Figur 3 dient der Verdeutlichung des Übergangsbereichs U. Hier werden wieder die beiden Kreisflächen der Figur 2 dargestellt, welche dem Dual-Energy-Bildbereich A∩B (innen) dem Single-Energy-Bildbereich A\B (außen) entsprechen. Dort wo diese beiden Bereiche aneinandergrenzen, würde in den späteren Daten zum Bremsvermögen $SPR_{DE}$ und $SPR_{SE}$ eine Nichtstetigkeit, ein "Sprung", erkennbar sein. Um diese Nichtstetigkeit der Daten zu kompensieren kann man sich einer Übergangsfunktion w(r) bedienen.

Der Übergangsbereich U ist hier innerhalb des Dual-Energy-Bildbereich A∩B angesiedelt (Rand des gestrichelten Kreises bis zum Rand des weißen Kreises) und hat die Breite Δ. Sei r eine Zahl zwischen 0 und Δ kann diese Übergangsfunktion w(r) die Wichtung des Einflusses der CT-Zahlen des Dual-Energy-Bildbereichs A∩B und des Single-Energy-Bildbereichs A\B auf das ermittelte effektive Bremsvermögen $SPR_{Eff}$ bestimmen.

Das effektive Bremsvermögen $SPR_{Eff}$ kann beispielsweise nach der Formel $SPR_{Eff}(r) = w(r) \cdot SPR_{DE} + (1-w(r)) \cdot SPR_{SE}$, mit den Bremsvermögen $SPR_{DE}$ und $SPR_{SE}$ und der Übergangsfunktion w(r) berechnet werden. Als eine einfache Übergangsfunktion w(r) kann beispielsweise w(r)=r-Δ angenommen werden, die einen linearen Übergang ergibt, wie hier angedeutet ist. Der Einfluss von $SPR_{DE}$ nimmt nach außen hin linear ab und der Einfluss von $SPR_{SE}$ nach außen hin linear zu.

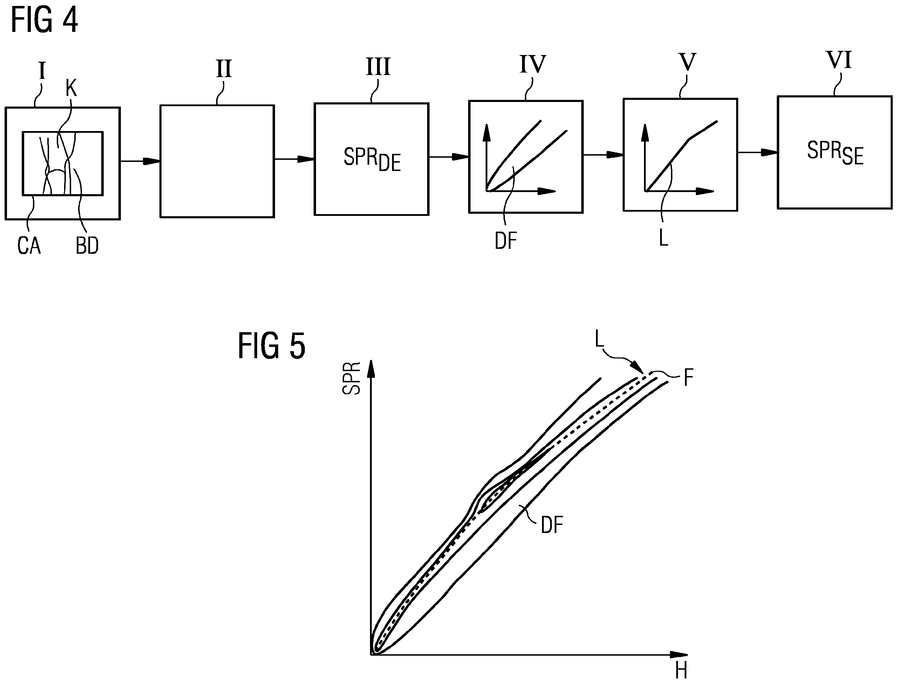

Figur 4 zeigt ein schematisches Blockbild des erfindungsgemäßen Verfahrens zur Ermittlung des Bremsvermögens SPR aus Computertomographie-Aufnahmen CA. Das Verfahren umfasst die folgenden Schritte.

In Schritt I erfolgt eine Bereitstellung von Computertomographie-Aufnahmen CA eines Körperbereichs K. Damit kann auch eine Anfertigung von Aufnahmen gemeint sein. Die Computertomographie-Aufnahmen CA werden dabei mit zwei unterschiedlichen Aufnahmeenergien A, B angefertigt bzw. bereitgestellt. Die Aufnahmen können Beispielsweise wie in Figur 2 skizziert wird, erfolgen.

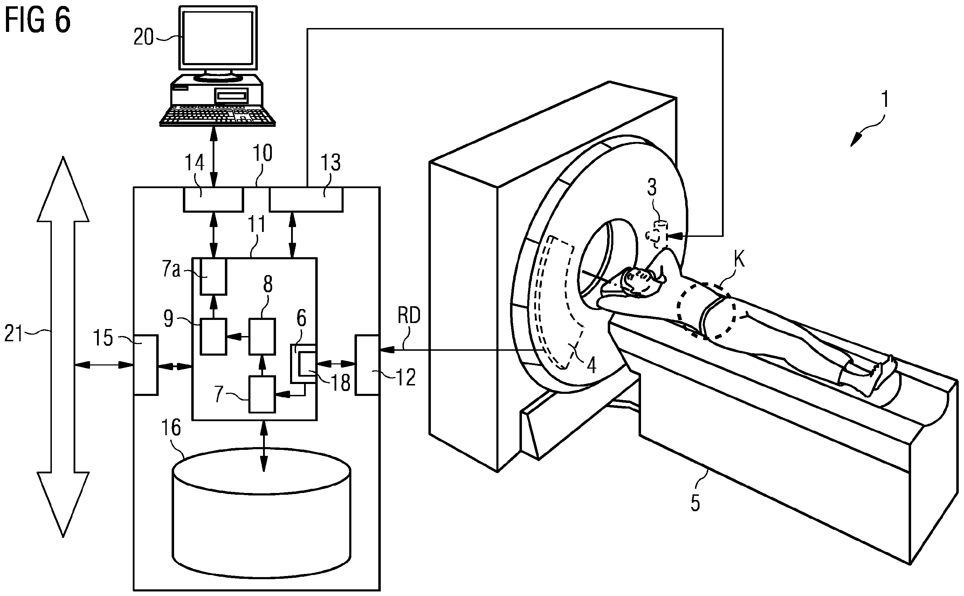

In Schritt II, der einen optionalen Schritt darstellt, wird eine Berechnung von CT-Zahlen H aus Bilddaten BD der Computertomographie-Aufnahmen CA vorgenommen. Dieser Schritt kann beispielsweise erforderlich sein, wenn die im vorangegangenen erwähnte Computertomographie-Aufnahmen CA in Form von Rohdaten dem Verfahren zur Verfügung gestellt werden und zunächst eine Bildrekonstruktion erfolgen muss, wie z.B. im Rahmen der Figur 6 genauer ausgeführt wird.

In Schritt III erfolgt eine Ermittlung des Bremsvermögens $SPR_{DE}$ für unterschiedliche CT-Zahlen H aus Dual-Energy-Bildbereichen A∩B der Computertomographie-Aufnahmen CA, also in denjenigen Bildbereichen in denen Bilddaten DB mit beiden Aufnahmeenergien A, B vorliegen.

In Schritt IV erfolgt die Bildung eines Datenfeldes DF, in dem die ermittelten Werte für das Bremsvermögen $SPR_{DE}$ für unterschiedliche CT-Zahlen H aus Dual-Energy-Bildbereichen A∩B in Abhängigkeit zu den CT-Zahlen H aufgetragen sind.

In Schritt V erfolgt die Bildung einer Nachschlagetabelle L, z.B. in Form einer Hounsfield-Look-Up-Tabelle (HLUT) aus dem Datenfeld DF. Dies kann beispielsweise bei einem Datenfeld DF, welches als Punktwolke vorliegt, dadurch erreicht werden, dass eine Funktion F an die Extrema der Punktwolke angepasst ("gefittet") wird. Diese Funktion F bestimmt dann die Werte der Nachschlagetabelle L.

In Schritt VI erfolgt eine Ermittlung des Bremsvermögens $SPR_{SE}$ für unterschiedliche CT-Zahlen H aus Single-Energy-Bildbereichen A\B der Computertomographie-Aufnahmen CA, also den Bildbereichen, in denen Bilddaten SB mit einer einzigen Aufnahmeenergie A, B vorliegen, aus der Nachschlagetabelle L.

Figur 5 zeigt ein mittels der Erfindung erstelltes Datenfeld DF, bei dem die durchgezogenen Linien Domänen einer Punktwolke unterschiedlicher Intensität andeuten sollen. In diese Punktewolke wurde eine Funktion F (gestrichelte Linie) eingepasst, welche die Nachschlagetabelle L definiert.

Figur 6 zeigt grob schematisch ein Computertomographiesystem 1 mit einer Steuereinrichtung 10 zur Durchführung des erfindungsgemäßen Verfahrens. Das Computertomographiesystem 1 weist in üblicher Weise einen Scanner 2 mit einer Gantry auf, in der eine Röntgenquelle 3 rotiert, die einen Patienten durchstrahlt, welcher mittels einer Liege 5 in einen Messraum der Gantry hineingeschoben wird, so dass die Strahlung auf einen der Röntgenquelle 3 jeweils gegenüberliegenden Detektor 4 trifft.

Das Computertomographiesystem 1 ist zur Aufnahme von Dual-Energy-Aufnahmen ausgelegt. Dabei ist es möglich, dass die Röntgenquelle 3 Strahlung mit zwei unterschiedlichen Energien erzeugen kann oder eine (hier nicht dargestellte) zweite Röntgenquelle mit einem zweiten Detektor vorhanden ist, z.B. 90° verdreht zu der vorgenannten Röntgenquelle 3. Es wird des Weiteren davon ausgegangen, dass die beiden Aufnahmen unterschiedlicher Aufnahmeenergie A, B mit unterschiedlichen Sichtfeldern $FoV_A$, $FoV_B$ (s. Fig. 2) erfolgen.

Es wird ausdrücklich darauf hingewiesen, dass es sich bei dem Ausführungsbeispiel gemäß Figur 6 nur um ein Beispiel eines CTs handelt und die Erfindung auch an beliebigen anderen CT-Konstruktionen, beispielsweise mit ringförmigem feststehendem Röntgendetektor und/oder mehreren Röntgenquellen genutzt werden kann.

Ebenso sind bei der Steuereinrichtung 10 nur die Komponenten dargestellt, die für die Erläuterung der Erfindung wesentlich oder hilfreich sind. Grundsätzlich sind derartige CT-Systeme und zugehörige Steuereinrichtungen dem Fachmann bekannt und brauchen daher nicht im Detail erläutert zu werden.

Eine Kernkomponente der Steuereinrichtung 10 ist hier ein Prozessor 11, auf dem verschiedene Komponenten in Form von Softwaremodulen realisiert sind. Die in dem Prozessor 11 dargestellten Komponenten entsprechen den Komponenten der erfindungsgemäßen Vorrichtung 11.

Die Steuereinrichtung 10 weist weiterhin eine Terminalschnittstelle 14 auf, an die ein Terminal 20 angeschlossen ist, über das ein Bediener die Steuereinrichtung 10 und somit das Computertomographiesystem 1 bedienen kann. Eine weitere Schnittstelle 15 ist eine Netzwerkschnittstelle zum Anschluss an einen Datenbus 21, um so eine Verbindung zu einem RIS (Radiologieinformationssystem) bzw. PACS PACS (Picture Archiving and Communication System = Bildarchivierungs- und Kommunikationssystem) herzustellen.

Über eine Steuerschnittstelle 13 kann von der Steuereinrichtung 10 der Scanner 2 angesteuert werden, d.h. es werden z.B. die Rotationsgeschwindigkeit der Gantry, die Verschiebung der Patientenliege 5 und die Röntgenquelle 3 selbst gesteuert. Über eine Akquisitionsschnittstelle 12 werden die Rohdaten RD aus dem Detektor 4 ausgelesen. Weiterhin weist die Steuereinrichtung 10 eine Speichereinheit 16 auf, in der z.B. Steuer- oder Messprotokolle hinterlegt sein können.

Als eine Softwarekomponente ist auf dem Prozessor 11 eine Schnittstelle 6 zum Empfang der Computertomographie-Aufnahmen CA eines Körperbereichs K mit zwei unterschiedlichen Aufnahmeenergien A, B ausgebildet. In diesem Beispiel empfängt die Schnittstelle Rohdaten RD, die zunächst aufbereitet werden müssen. Dazu ist in der Schnittstelle 6 eine Bilddaten-Rekonstruktionseinheit 18 implementiert, mit welcher aus den über die Datenakquisitions-Schnittstelle 12 erhaltenen Rohdaten RD die gewünschten Bilddaten rekonstruiert werden. Diese Bilddaten-Rekonstruktionseinheit 18 verarbeitet die Rohdaten auf eine Weise, dass die entstehenden Bilddaten den CT-Zahlen H entsprechen oder diese zumindest enthalten.

Die rekonstruierten Computertomographie-Aufnahmen CA werden an eine erste Ermittlungseinheit 7 weitergegeben. Diese ermittelt das Bremsvermögen $SPR_{DE}$ für unterschiedliche CT-Zahlen H aus Dual-Energy-Bildbereichen A∩B der Computertomographie-Aufnahmen CA,

Die ermittelten Daten werden an eine Recheneinheit 8 weitergegeben. Diese bildet ein Datenfeld DF, in dem die ermittelten Werte für das Bremsvermögen $SPR_{DE}$ für unterschiedliche CT-Zahlen H aus Dual-Energy-Bildbereichen (A∩B) in Abhängigkeit zu den CT-Zahlen H aufgetragen sind.

Aus diesem Datenfeld DF erstellt ein Nachschlagetabellen-Modul 9 eine Nachschlagetabelle L.

Zuletzt ermittelt eine zweite Ermittlungseinheit 7a das Bremsvermögen $SPR_{SE}$ für unterschiedliche CT-Zahlen H aus Single-Energy-Bereichen A\B der Computertomographie-Aufnahmen CA aus der Nachschlagetabelle.

Die ermittelten Werte für die jeweiligen Bremsvermögen $SPR_{DE}$ und $SPR_{SE}$ können dann auf dem Terminal 20 ausgegeben werden, in der Speichereinheit 16 abgespeichert werden oder mittels des Datenbusses 21 anderen Geräten zur Verfügung gestellt oder in einem Netzwerk-Speichereinheit abgespeichert werden.

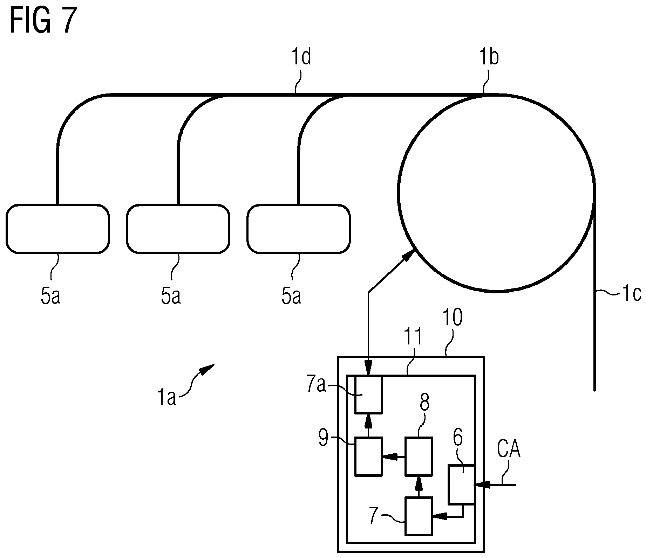

Figur 7 zeigt eine grob schematische Darstellung eines Partikeltherapiesystems 1a mit einem Ausführungsbeispiel einer erfindungsgemäßen Steuereinrichtung 10 zur Durchführung des Verfahrens. Das Partikeltherapiesystem 1a ist hier als Beschleunigersystem dargestellt und umfasst drei Behandlungsplätze 5a. Über die im Bild rechts dargestellte Beamline 1c werden vorbeschleunigte Partikel, z.B. Protonen, in einem Beschleunigerring 1b eingebracht, dort auf Endgeschwindigkeit beschleunigt und ggf. gespeichert. Über eine Extraktions-Beamline 1d werden die Partikel zu den Behandlungsplätzen 5a gelenkt und stehen dort zur Behandlung von Patienten bereit.

Die Partikelenergie muss dabei exakt gesteuert werden, damit sie ihre maximale Wirkung an einem klar definierten Ort im Patienten entfalten. Zur Bestimmung dieser Energie ist das Bremsvermögen in dem entsprechenden Körperbereich K des Patienten ein wichtiges Maß.

Die Steuereinrichtung 10, in deren Prozessor 11 die erfindungsgemäße Vorrichtung, wie bereits in Figur 6 genauer erklärt, ausgeformt ist, steuert z.B. die Energie des Beschleunigerrings 1b und die Extraktion über die Extraktions-Beamline 1d. Die erfindungsgemäße Vorrichtung im Prozessor 11 kann aus geeigneten Computertomographieaufnahmen CA das Bremsvermögen in dem betreffenden Körperbereich K bestimmen und die Steuereinrichtung 10 aus diesem Bremsvermögen die geeignete Beschleunigerenergie ermitteln und den Beschleuniger entsprechend steuern. Generell ist es von Vorteil, wenn das Bremsvermögen so exakt wie möglich bekannt ist. Je genauer das Bremsvermögen bestimmt werden kann, desto genauer ist die Beplanung was direkt Auswirkungen auf die Effektivität der Bestrahlung hat.

Es wird abschließend noch einmal darauf hingewiesen, dass es sich bei den vorhergehend detailliert beschriebenen Verfahren sowie bei dem dargestellten Computertomographiesystem 1 und dem Partikeltherapiesystem 1a lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriff "Einheit" und "Modul" nicht aus, dass die betreffenden Komponenten aus mehreren zusammenwirkenden TeilKomponenten bestehen, die gegebenenfalls auch räumlich verteilt sein können.

**Patentansprüche**

1. Verfahren zur Ermittlung des Bremsvermögens SPR aus Computertomographie-Aufnahmen (CA), umfassend die Schritte:

- Bereitstellung von Computertomographie-Aufnahmen (CA) eines Körperbereichs (K) mit zwei unterschiedlichen Aufnahmeenergien (A, B),
- Ermittlung des Bremsvermögens $SPR_{DE}$ für unterschiedliche CT-Zahlen H aus Dual-Energy-Bildbereichen (A∩B) der Computertomographie-Aufnahmen (CA), und
- Bildung eines Datenfeldes (DF), in dem die ermittelten Werte für das Bremsvermögen $SPR_{DE}$ für unterschiedliche CT-Zahlen H aus Dual-Energy-Bildbereichen (A∩B) in Abhängigkeit zu den CT-Zahlen H aufgetragen sind,
- Bildung einer Nachschlagetabelle (L) aus dem Datenfeld (DF),
- Ermittlung des Bremsvermögens $SPR_{SE}$ für unterschiedliche CT-Zahlen H aus Single-Energy-Bildbereichen (A\B) der Computertomographie-Aufnahmen (CA) mittels der Nachschlagetabelle (L).

2. Verfahren nach Anspruch 1, wobei das Datenfeld (DF) als Punktwolke, bevorzugt in Form eines Histogramms, vorliegt und eine Funktion (F), welche Werte der Nachschlagetabelle (L) definiert, an die Punktwolke angepasst wird.

3. Verfahren nach einem der vorangehenden Ansprüche, wobei für eine Anzahl von Voxeln einer Computertomographie-Aufnahme (CA) mit bekannter Bildposition ein ortsabhängiges Bremsvermögen $SPR_{DE}$ und/oder $SPR_{SE}$ ermittelt wird.

4. Verfahren nach einem der vorangehenden Ansprüche, wobei für unterschiedliche Durchmesser von aufgenommenen Regionen unterschiedliche Nachschlagetabellen (L) erstellt werden.

5. Verfahren nach Anspruch 4, wobei für eine Nachschlagetabelle (L) der betreffende Durchmesser aus der in einer zugehörigen Computertomographie-Aufnahmen (CA) abgebildeten Region ermittelt oder geschätzt werden.

6. Verfahren nach Anspruch 4 oder 5, wobei zur Ermittlung des Bremsvermögens $SPR_{SE}$ für eine CT-Zahl H im Single-Energy-Bereich (A\B) der in diesem Bereich vorliegende Durchmesser geschätzt wird und sich das Bremsvermögen $SPR_{SE}$ aus einer linearen Interpolation von Nachschlagetabellen (L) ergibt.

7. Verfahren nach einem der vorangehenden Ansprüche, wobei vor der Ermittlung der Werte des Bremsvermögens $SPR_{DE}$ eine Korrektur einer Strahlaufhärtung erfolgt, wobei bevorzugt zumindest eine dedizierte Kalibration erfolgen, um eine dedizierte Nachschlagetabelle (L) zu erzeugen.

8. Verfahren nach einem der vorangehenden Ansprüche, wobei im Falle aneinandergrenzender Dual-Energy-Bildbereiche (A∩B) und Single-Energy-Bildbereiche (A\B) in einem vorbestimmten Übergangsbereich (U), welcher eine Dicke Δ aufweist, aus dem Bremsvermögen $SPR_{DE}$ des Dual-Energy-Bildbereichs (A∩B) und dem Bremsvermögen $SPR_{SE}$ des Single-Energy-Bildbereichs (A\B) und einer vorbestimmten Übergangsfunktion w(r) mit dem Ortsparameter r, mit 0<r<Δ, ein effektives Bremsvermögen $SPR_{Eff}$ berechnet wird.

9. Verfahren nach Anspruch 8, wobei sich das effektive Bremsvermögen $SPR_{Eff}$ mit den Bremsvermögen $SPR_{DE}$ und $SPR_{SE}$ und der Übergangsfunktion w(r) nach der Formel

$$SPR_{Eff}(r) = w(r) \cdot SPR_{DE} + (1 - w(r)) \cdot SPR_{SE}$$

berechnet.

10. Vorrichtung zur Ermittlung des Bremsvermögens SPR aus Computertomographie-Aufnahmen (CA), umfassend

- eine Schnittstelle (6) zum Empfang von Computertomographie-Aufnahmen (CA) eines Körperbereichs (K) mit zwei unterschiedlichen Aufnahmeenergien (A, B);
- eine erste Ermittlungseinheit (7) zur Ermittlung des Bremsvermögens $SPR_{DE}$ für unterschiedliche CT-Zahlen H aus Dual-Energy-Bildbereichen (A∩B) der Computertomographie-Aufnahmen (CA);
- eine Recheneinheit (8) zur Bildung eines Datenfeldes (DF), in dem die ermittelten Werte für das Bremsvermögen $SPR_{DE}$ für unterschiedliche CT-Zahlen H aus Dual-Energy-Bildbereichen (A∩B) in Abhängigkeit zu den CT-Zahlen H aufgetragen sind;
- ein Nachschlagetabellen-Modul (9) zur Bildung einer Nachschlagetabelle (L) aus dem Datenfeld (DF);
- eine zweite Ermittlungseinheit (7a) zur Ermittlung des Bremsvermögens $SPR_{SE}$ für unterschiedliche CT-Zahlen H aus Single-Energy-Bereichen (A\B) der Computertomographie-Aufnahmen (CA) mittels der Nachschlagetabelle.

11. Steuereinrichtung (10) für ein Computertomographiesystem (1) oder ein Partikeltherapiesystem (1a), welche zur Durchführung eines Verfahrens nach einem der Ansprüche 1 bis 9 ausgelegt ist und/oder eine Vorrichtung nach Anspruch 10 umfasst.

12. Computertomographiesystem (1) umfassend eine Steuereinrichtung (10) nach Anspruch 11.

13. Partikeltherapiesystem (1a) umfassend eine Steuereinrichtung (10) nach Anspruch 11.

14. Computerprogrammprodukt mit einem Computerprogramm, welches direkt in eine Speichereinrichtung einer Steuereinrichtung (10) oder einer Rechenvorrichtung ladbar ist, mit Programmabschnitten, um alle Schritte des Verfahrens nach einem der Ansprüche 1 bis 9 auszuführen, wenn das Computerprogramm in der Steuereinrichtung (10) oder der Rechenvorrichtung ausgeführt wird.

15. Computerlesbares Medium, auf welchem von einer Rechnereinheit einlesbare und ausführbare Programmabschnitte gespeichert sind, um alle Schritte des Verfahrens nach einem der Ansprüche 1 bis 9 auszuführen, wenn die Programmabschnitte von der Rechnereinheit ausgeführt werden.

**Claims**

1. Method for determining the stopping power SPR from computed tomography scans (CA), comprising the steps:

- Preparation of computed tomography scans (CA) of a body region (K) with two different recording energies (A, B),
- Determination of the stopping power $SPR_{DE}$ for different CT numbers H from dual energy image areas (A∩B) of the computed tomography scans (CA), and
- Formation of a data field (DF) in which the determined values for the stopping power $SPR_{DE}$ for different CT numbers H from dual energy image areas (A∩B) are applied as a function of the CT numbers H,
- Formation of a look-up table (L) from the data field (DF),
- Determination of the stopping power $SPR_{SE}$ for different CT numbers H from single energy image areas (A\B) of the computed tomography scans (CA) by means of the look-up table (L).

2. Method according to claim 1, wherein the data field (DF) exists as a point cloud, preferably in the form of a histogram, and a function (F) which defines values of the look-up table (L) is adapted to the point cloud.

3. Method according to one of the preceding claims, wherein a location-dependent stopping power $SPR_{DE}$ and/or $SPR_{SE}$ is determined for a number of voxels of a computed tomography scan (CA) with known image position.

4. Method according to one of the preceding claims, wherein different look-up tables (L) are created for different diameters of scanned regions.

5. Method according to claim 4, wherein the relevant diameters for a look-up table (L) are determined or estimated from the region depicted in an associated computed tomography scan (CA).

6. Method according to claim 4 or 5, wherein for the determination of the stopping power $SPR_{SE}$ for a CT number H in the single energy range (A\B) the diameter in this range is estimated and the stopping power $SPR_{SE}$ results from a linear interpolation of look-up tables (L).

7. Method according to one of the preceding claims, wherein before the determination of the values of the stopping power $SPR_{DE}$ a correction of beam hardening takes place, wherein preferably at least one dedicated calibration takes place to generate a dedicated look-up table (L).

8. Method according to one of the preceding claims, wherein in the case of adjoining dual energy image areas (A∩B) and single energy image areas (A\B) in a predetermined transition area (U) with a thickness Δ, an effective stopping power $SPR_{Eff}$ is calculated from the stopping power $SPR_{DE}$ of the dual energy image area (A∩B) and the stopping power SPRSE of the single energy image area (A\B) and a predetermined transition function w(r) with the location parameter r, with 0<r<Δ.

9. Method according to claim 8, wherein the effective stopping power $SPR_{Eff}$ is calculated with the stopping power $SPR_{DE}$ and $SPR_{SE}$ and the transition function w(r) according to the formula

$$SPR_{Eff}(r) = w(r) \cdot SPR_{DE} + (1 - w(r)) \cdot SPR_{SE} \, .$$

10. Device for determining the stopping power SPR from computed tomography scans (CA), comprising

- An interface (6) for receiving computed tomography scans (CA) of a body region (K) with two different recording energies (A, B);
- A first determination unit (7) for determining the stopping power $SPR_{DE}$ for different CT numbers H from dual energy image areas (A∩B) of the computed tomography scans (CA);
- A computing unit (8) for forming a data field (DF) in which the determined values for the stopping power $SPR_{DE}$ for different CT numbers H from dual energy image areas (A∩B) are applied as a function of the CT numbers H;
- A look-up table module (9) for forming a look-up table (L) from the data field (DF);
- A second determination unit (7a) for determining the stopping power $SPR_{SE}$ for different CT numbers H from single energy areas (A\B) of the computed tomography scans (CA) by means of the look-up table.

11. Control device (10) for a computed tomography system (1) or a particle therapy system (1a) which is designed to execute a method according to one of claims 1 to 9 and/or comprises a device according to claim 10.

**12.** Computed tomography system (1) comprising a control device (10) according to claim 11.

**13.** Particle therapy system (1a) comprising a control device (10) according to claim 11.

**14.** Computer program product with a computer program which can be directly loaded into a storage device of a control device (10) or a computing device, with program segments to perform all the steps of the method according to one of claims 1 to 9, if the computer program is performed in the control device (10) or the computing device.

**15.** Computer-readable medium on which program segments readable and executable by a processor unit are stored to perform all the steps of a method according to one of claims 1 to 9 if the program segments are performed by the processor unit.

**Revendications**

**1.** Procédé de détermination du pouvoir SPR de freinage à partir d'enregistrements (CA) de tomographie par ordinateur, comprenant les stades :

- on se procure des enregistrements (CA) de tomographie par ordinateur d'une partie (K) du corps à deux énergies (A, B) d'enregistrement différentes,
- on détermine le pouvoir $SPR_{DE}$ de freinage de nombres-CT H différents de parties (A∩B) d'image à énergie duale des enregistrements (CA) de tomographie par ordinateur, et
- on forme un champ (DF) de données, dans lequel les valeurs déterminées des pouvoirs $SPR_{DE}$ de freinage de nombres-CT H différents de parties (A∩B) d'image à énergie duale sont portées en fonction des nombres-CT H,
- on forme une table (L) de références à partir du champ (DF) de données,
- on détermine le pouvoir $SPR_{SE}$ de freinage pour des nombres-CT H différents à partir de parties (A\B) d'image à énergie unique des enregistrements (CA) de tomographie par ordinateur au moyen de la table (L) de référence.

**2.** Procédé suivant la revendication 1, dans lequel le champs (DF) de données se présente sous la forme d'un nuage de points, de préférence sous la forme d'un histogramme, et on adapte une fonction (F), qui définit des valeurs de la table (L) de référence au nuage de points.

**3.** Procédé suivant l'une des revendications précédentes, dans lequel, pour un nombre de voxels d'un enregistrement (CA) de tomographie par ordinateur, ayant une position d'image connue, on détermine un pouvoir $SPR_{DE}$ et/ou $SPR_{SE}$ de frein en fonction de l'emplacement.

**4.** Procédé suivant l'une des revendications précédentes, dans lequel on établit des tables (L) de référence différentes pour des diamètres différents de régions enregistrées.

**5.** Procédé suivant la revendication 4, dans lequel, pour une table (L) de référence, on détermine ou on évalue le diamètre concerné à partir de la région reproduite dans un enregistrement (CA) de tomographie par ordinateur associé.

**6.** Procédé suivant la revendication 4 ou 5, dans lequel, pour déterminer le pouvoir $SPR_{SE}$ de freinage pour un nombre-CT H dans une partie (A\B) à énergie unique, on estime le diamètre présent dans cette partie et on obtient le pouvoir $SPR_{SE}$ de freinage par une interpolation linéaire de tables (L) de référence.

**7.** Procédé suivant l'une des revendications précédente, dans lequel, avant la détermination des valeurs du pouvoir $SPR_{DE}$ de freinage, on effectue une correction d'un durcissement de faisceau, en effectuant de préférence au moins un étalonnage dédié, afin de produire une table (L) de référence dédiée.

**8.** Procédé suivant l'une des revendications précédentes, dans lequel, dans le cas de parties (A∩B) d'images à énergie duale voisines les unes des autres et de parties (A\B) d'image à énergie unique dans une partie (U) de transition déterminée à l'avance, qui a une épaisseur $\Delta$, on calcule, à partir du pouvoir $SPR_{DE}$ de freinage de la partie (A∩B) d'image à énergie duale et du pouvoir $SPR_{SE}$ de freinage de la partie (A\B) d'image à énergie unique et d'une fonction w(r) déterminée à l'avance, ayant le paramètre r d'emplacement, avec $0<r<\Delta$, un pouvoir $SPR_{Eff}$ de freinage efficace.

9.  Procédé suivant la revendication 8, dans lequel on calcule le pouvoir $SPR_{Eff}$ de freinage efficace par les pouvoirs $SPR_{DE}$ et $SPR_{SE}$ de freinage et la fonction w(r) de transition suivant la formule

$$SPR_{Eff}(r) = w(r) \, SPR_{DE} + (1 - w(r)) \, SPR_{SE}$$

10. Dispositif de détermination du pouvoir SPR de freinage à partir d'enregistrements (CA) de tomographie par ordinateur, comprenant

- une interface (6) de réception d'enregistrements (CA) de tomographie par ordinateur d'une partie (K) du corps à deux énergies (A, B) d'enregistrement différentes ;
- une première unité (7) de détermination pour déterminer le pouvoir $SPR_{DE}$ de freinage pour des nombres CT H différents à partir de parties (A∩B) d'image à énergie duale des enregistrements (CA) de tomographie par ordinateur ;
- une unité (8) de calcul pour former un champ (DF) de données, dans lequel les valeurs déterminées du pouvoir $SPR_{DE}$ de freinage pour des nombres-CT H différents à partir de parties (A∩B) d'image à énergie duale sont portées en fonction des nombres-CT H ;
- un module (9) de table de référence pour former une table (L) de référence à partir du champ (DF) de données ;
- une deuxième unité (7a) de détermination pour déterminer le pouvoir $SPR_{SE}$ de freinage pour des nombres-CT H différents à partir des parties (A\B) à énergie unique des enregistrements (CA) de tomographie par ordinateur au moyen de la table de référence.

11. Dispositif (10) de commande d'un système (1) de tomographie par ordinateur ou d'un système (1a) de thérapie par particules, qui est conçu pour effectuer un procédé suivant l'une des revendications 1 à 9 et/ou qui comprend un dispositif suivant la revendication 10.

12. Système (1) de topographie par ordinateur, comprenant un dispositif (10) de commande suivant la revendication 11.

13. Système de thérapie (1a) par particules, comprenant un dispositif (10) de commande suivant la revendication 11.

14. Produit de programme d'ordinateur, comprenant un programme d'ordinateur, qui peut être chargé directement dans un dispositif de mémoire d'un dispositif (10) de commande ou dans un dispositif de calcul, comprenant des parties de programme pour effectuer tous les stades du procédé suivant l'une des revendications 1 à 9, lorsque le programme d'ordinateur est réalisé dans le dispositif (10) de commande ou dans le dispositif de calcul.

15. Support déchiffrable par ordinateur, sur lequel sont mises en mémoire des parties de programme déchiffrables et réalisables par une unité de calcul, afin d'effectuer tous les stades du procédé suivant l'une des revendications 1 à 9, lorsque les parties de programme sont réalisées par l'unité de calcul.

FIG 1   STAND DER TECHNIK

FIG 2

FIG 3

FIG 4

FIG 4

I — K, CA, BD
II
III — SPR$_{DE}$
IV — DF
V — L
VI — SPR$_{SE}$

FIG 5

SPR, L, F, DF, H

EP 3 511 053 B1

FIG 6

EP 3 511 053 B1

FIG 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **N. HÜNEMOHR.** Ion Range estimation by using dual energy computed tomography. *Z. Med. Phys.,* 2013, vol. 23, 300-313

- **N. HÜNEMOHR.** Experimental verification of ion stopping power prediction from dual energy CT data in tissue surrogates. *Phys. Med. Biol.,* 2014, vol. 59, 83-96